# EUROPEAN PATENT APPLICATION

(11) **EP 1 132 044 A1**
(43) Date of publication of application: **12.09.2001**
(21) Application number: 01200853.8
(22) Date of filing: 06.03.2001
(51) Int. Cl.: A61B 5/0215, A61B 5/03

(54) **A device for providing an indication concerning the pressure existing in an environment**

(30) Priority: 06.03.2000 NL 1014566
(71) Applicant: B.V. Beleggings en Exploitatie Maatschappij Elsvege, 7535 PG Enschede (NL)
(72) Inventor: Henderikx, Hubertus Henricus Maria, 6039 RG Stramproy (NL); Glastra, Hendrik, 7535 PG Enschede (NL)
(74) Representative: Timmers, Cornelis Herman Johannes

(57) **Abstract**

A device for providing an indication concerning the pressure existing in an environment, particularly in human or animal tissue, with a closed capsule (4) to be implanted therein, said capsule (4) having a wall (6) which is partly flexible and being partly filled with a quantity of material (12) which is displaced as a result of movements of the wall (6) and of which, in a reference position of the capsule (4), the surface level (14) with respect to one or more radiopaque markers (10) on the capsule (4) can be detected, so that by means of repeated observations changes in the tissue can be detected.

## Description

The invention relates to a device for providing an indication concerning the pressure existing in an environment.

The measurement of the pressure in an environment, for instance in a liquid or viscous environment is no problem when it is possible to introduce therein from the outside a pressure sensor. Monitoring such a pressure from a distance is also no problem when one can use in this environment a pressure sensor supplying signals, representing the pressure, which can be transmitted wirelessly. However measuring the pressure which is present in a body environment, for instance in an existing or developing tissue in a human or animal body, using an element to be implanted therein and which can supply outside the body an indication about the pressure in this environment has until now not been realised in practice. A wireless measurement of a quantity by means of a sensor, implanted at a predetermined position in the human body, for instance in a blood vessel and, particularly, in the neighbourhood of an aneurism has, as has been demonstrated, the drawback that it is practically impossible to transmit the signals, produced by this sensor, to outside the body. As a result of the very limited electrical power which is available in an implanted sensor the output signal thereof will be very weak indeed. Practice has shown that the screening action of the surround tissues is so strong that, the more as a result of the always present electrical noise fields no usuable signal can be detected outside the body.

Yet a local monitoring of the pressure, present in the tissue which is developing at or near an aneurism, particularly after the placing of a stent in the aneurism, is of crucial importance for an early discovery of anomalities and complications. These can develop unobserved by the patient into a complication which must be treated by surgery. This is, however, particularly in case of an aorta aneurism, a very serious operation which no surgeon will undertake in an early stage but, as said, complications can develop so fast that surgery, when undertaken, comes too late.

Thus there is a great need for an implantable element by means of which the pressure in an environment, particularly the tissue as already formed or to be formed in the neighbourhood of an aneurism, can be monitored.

The invention provides such a device and proposes that this consists of a closed capsule, to be influenced by this pressure of which the wall is, at least partially, flexible and which is partly filled by a quantity of material which is displaced as a result of movements of the wall, and of which, in a reference position of the capsule, the surface level with respect to one or more markers on the capsule, can be detected.

In this capsule the surface level in the reference position, for instance the position in which the patient is standing up, will have a certain position with respects to the markers. When the pressure surrounding a capsule increases the position of the surface level with respect to the markers will change, particularly will rise. When the filling material and/or the markers consist of a material which does not transmit X-rays or transmit then only partially, a simple X-ray examination will immediately show that the pressure in the tissue is changing, and particularly is rising.

The filling material can be contrast liquid or can also exist of a material with very fine grains, which flows easily.

In a preferred embodiment at least a part of the wall of the capsule is X-ray transmissive while behind this wall part a fixed element with markers is provided. Particularly the capsule is in its rest position pear-shaped with the fixed element positioned along the longest axis thereof.

The implantation of the capsule can be done by means of the usual implantating techniques (catheter and removable shield). The capsule can also be fixed to a stent and the whole is then implanted in one treatment.

It is observed that the French patent specification FR 1.178.475 describes a device for measuring the pressure in a fluid, comprising a capsule with at least one flexible wall part (membrane) and filled with a liquid, the lumen of said capsule being connected to a tube with small diameter, the liquid level therein depending on the pressure exerted on the membrane. It is clear that such a device is totally unsuited for implantation in a human or animal body.

The invention is elucidated on the hand of the drawing. Herein shows:
fig. 1 a schematic cross-section of the capsule of the device according to the invention in the non-loaded position,
fig. 2 a cross-section similar to the one according to fig. 1 but now in the state in which from all sides a certain pressure is excerted on the capsule,
fig. 3 schematically the way in which the device according to the invention is accommodated in body tissue.

An embodiment of the device according to the invention, in its whole denoted with reference numeral 2 in the drawings, comprises a capsule 4 which, in uninfluenced state, shows a essentially pear-shaped cross-section and is made up from a flexible wall 6 and a longitudinal, fixed, rod-shaped element 8. This rod-shaped element 8 carries markers 10 which are not transmissive for X-rays, being stripes of a radiopaque material (gold, titanium, barium).

The capsule 4 is partly filled with flowing material 12, which can be a contrastliquid or consist of very fine, well-flowing, grains.

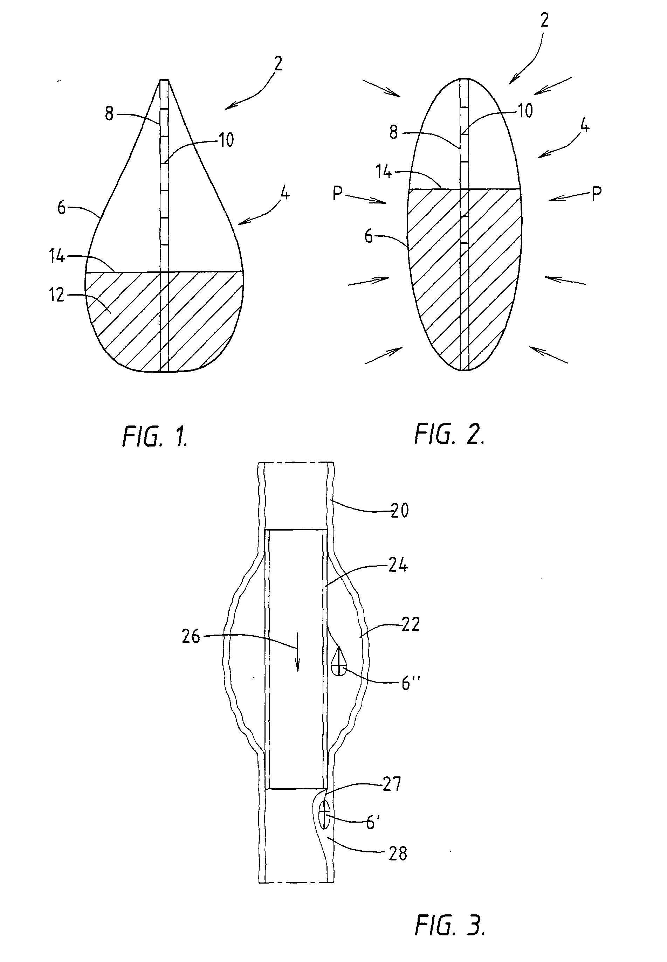

As fig. 1 shows the whole is in its uninfluened state nearly pear-shaped. However when, as fig. 2 schematically shows, a pressure is excerted on the wall of the capsule 4, this capsule will obtain a more or less flattened shape and as a result of the fact that the volume enclosed by the wall 6 remains the same the level 14 of the filling material 12 will rise. Thus this level 14 will have a different position with respect to the markers 10.

By means of a simple irradiation with X-rays the change of the level 14 with respect to the markers 10 can be detected, giving an indication with respect to the change of the pressure acting upon the capsule 4.

Fig. 3 shows very schematically a blood vessel 20 in which is formed an aneurism 22, thus a local widening and weakening of the vesell wall. This aneurism 22 has, in the usual way, bridged by an implanted stent 24. Practice has shown that, after the implantation of a stent such as the stent 24, downstream, thus in the direction of the arrow 26 which indicates the direction of the blood flow, parasitic tissue develops, such as indicated with 28. To monitor this development it is of importance to know the pressure which reigns within this developed tissue 28, and to this end within this tissue a capsule 6', such as described on the hand of fig. 1 and 2, is implanted. This capsule can be fixed to the stent 24 by means of the wire 27. The implantation of an independent, single, stent can be done using the usual techniques; when the capsule is anchored to a stent both are implanted in one step.

The way in which the pressure within the tissue 28 changes, and particularly increases, is an indication for the tissue-growth and it is of great importance to monitor the evolution of the pressure. By means of the device as herein-above described, taking of X-ray images at regular intervals makes it possible to monitor the development of this tissue growth, an achievement which was, until now, impossible.

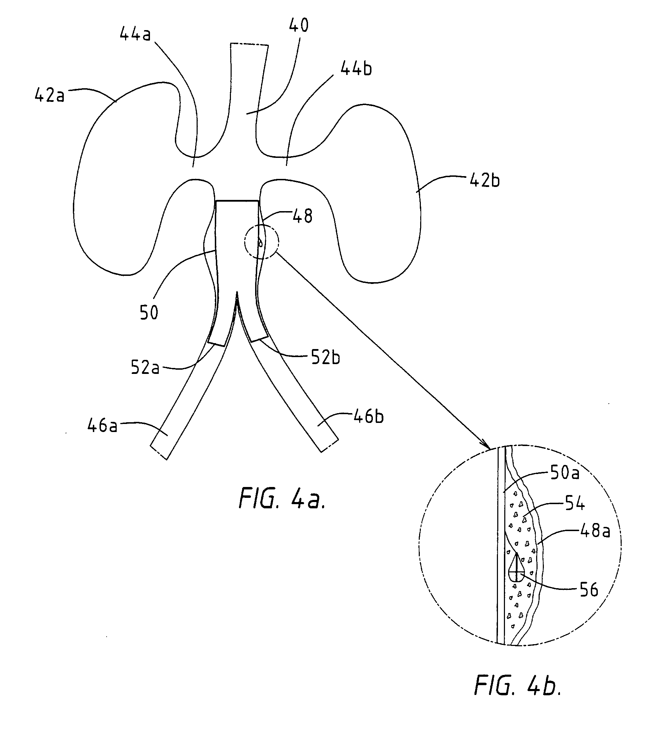

With particular advantages the invention is used to monitor a usual aorta prothesis, such as schematically shown in the fig. 4a and 4b. Fig. 4a shows an aorta 40 with the vena renalis 44a, 44b leading to the kidneys 42a and 42b on the one hand, and the arteria iliaca 46a, 46b on the other hand, and between them a very well developed aneurism 48 supported by a prothesis 50 with the two "pipes" 42a, 42b. A capsule 56, implanted in the developing, gel-like tissue 54 which is present between the aorta wall 48a and the stent wall 50a and anchored by the wire 58 to this stent makes it possible in the way as described above to monitor the development of this growth. The capsule 56 is placed into position at the same time as the stent 50 by means of the usual techniques.

Of course within the scope of the invention, as determined by the claims, many embodiments are possible, particularly with regard to the shape of the capsule and the way in which the markers are provided.

## Claims

1. Device for providing an indication concerning the pressure existing in an environment, **characterized by** a closed capsule, to be influenced by this pressure, of which the wall is at least partly flexible and which is partly filled with a quantity of material which can be displaced as a result of movements of the wall and of which, in a reference position of the capsule, the surface level with regard to one or more markers on the capsule can be detected.

2. Device according to claim 1, **characterized in that** the filling material and the markers consist of a material which does not or only partly transmit X-rays.

3. Device according to claim 2, **characterized in that** the filling material is a contrastliquid.

4. Device according to claim 2, **characterized in that** the filling material is a very fine-grain material.

5. Device according to claims 2-4, **characterized in that** of the capsule at least part of the wall is transmissive for X-rays and that a fixed element with markers is provided behind this wall part.

6. Device according to claim 5, **characterized in that** the capsule is in its unloaded state essentially pear-shaped, the fixed element being positioned along the long axis thereof.

7. Device according to claims 1-6, **characterized by** means for anchoring it to a stent.
